# EUROPEAN PATENT APPLICATION

(11) **EP 1 625 859 A1**
(43) Date of publication of application: **15.02.2006**
(21) Application number: 04077289.9
(22) Date of filing: 13.08.2004
(51) Int. Cl.: A61L 2/00, A61L 2/08, A61K 31/43

(54) **A process for the preparation of a package comprising a sterilised bulk of a substance, and a package comprising a sterilised bulk of a penicilin**

(71) Applicant: Access Group ApS, 3230 Graested (DK)
(72) Inventor: Klose, Bruno, 34431 Marsberg (DE)
(74) Representative: Inspicos A/S

(57) **Abstract**

The invention provides a process for the preparation of a package comprising a sterilised bulk of a substance, e.g. a drug substance such as penicillins (e.g. amoxicillin and ampicillin), a food product, a food ingredient, and a chemical, the process includes: providing the bulk of the substance; packing said bulk in a primary packing means and sealing said primary packing means, the void space in said primary packing means essentially consisting of an unreactive gas (e.g. carbon dioxide), thereby forming a package comprising said bulk of the substance; and exposing said package to electron treatment, thereby obtaining a package comprising a sterilized bulk of the substance. The invention also provides a package comprising a sterilized drug substance in a sealed primary packing means, said drug substance being selected from penicillins (e.g. amoxicillin and ampicillin), the void space of primary packing means essentially consisting of an unreactive gas.

## Description

### FIELD OF THE INVENTION

The present invention relates to an improved process for the preparation of a package comprising a sterilised bulk of a substance, e.g. a drug substance, such as a penicillin, in particular amoxicillin or ampicillin, a food product, a food ingredient or a chemical. The invention also relates to a package comprising a sterilized bulk of a penicillin.

### BACKGROUND OF THE INVENTION

Due to high molecular tension in the molecules, penicillins have a disposition for degradation. The exposure of energy so as to sterilise a bulk of a penicillin may lead to oxidative and disintegrative products. The disintegration process is a result of vibration of the molecular bonds. There are three main factors responsible for degradation: the activation energy, the time of exposure, and the environment (in particular oxygen). Under standard environment conditions (light, oxygen), the result will be a high rate of degradation and oxidation. Upon degradation, the colour of the bulk (often a powder or a granulate material) turns from white to yellow up to brown. This is of course unacceptable from an aesthetic point of view, but also because the general quality of the product may be affected.

The above-mentioned problems are also known from a number of other substances for which sterilization is necessary, e.g. food product (such as milk powder), food ingredients, and chemicals.

This being said, there is a need for an efficient process for sterilisation of bulk substances such as bulk drug substances, bulk food products, bulk food ingredients, and bulk chemicals.

### BRIEF DESCRIPTION OF THE FIGURE

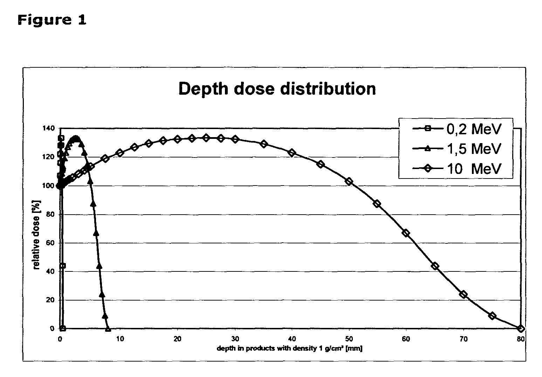

Figure 1 shows the penetration depth of electron treatment.

### DESCRIPTION OF THE INVENTION

The present invention provides a process for the preparation of a package comprising a sterilised bulk of a substance, e.g. a bulk of a drug substance, said process comprising the following steps:
a) providing the bulk of the substance;
b) packing said bulk in a primary packing means and sealing said primary packing means, the void space in said primary packing means essentially consisting of an unreactive gas, thereby forming a package comprising said bulk of the substance; and
c) exposing said package to electron treatment, thereby obtaining a package comprising a sterilized bulk of the substance.

### The bulk of a substance

The process of the invention may in principle be used for any substance for which it is required that the substance is to be presented in a packed, sterile form. Thus, the invention is particularly relevant for drug substances, especially penicillins, for food products (e.g. milk powder), for food ingredients, and for chemicals in general.

Penicillins represent a diverse group of isomeric and closely related naturally occurring and synthetic antibiotics with outstanding antibacterial activity. Illustrative examples of penicillins are amoxicillin, ampicillin, penicillin G procaine, cephalosporin, erythromycin, neomycin, streptomycin, tetracycline, and gentamicin. In the present context, amoxicillin and ampicillin are found to be particularly interesting.

The terms "bulk of a substance", "bulk of the substance" and "bulk" are used synonymously.

The bulk substance is preferably presented in substantially dry form, e.g. with at water content of at the most 4%, in particular at the most 1%.

### Sterile

When used herein, the terms "sterile" and "sterilized" are intended to mean that the bulk of the substance in question is essentially free of viable micro-organisms according to Ph. Eur. 2.6.1. Products which are produced under controlled fabrication conditions may be contaminated with micro-organism, and it is then necessary to sterilize the product in order to obtain a suitable sterility assurance level.

During the sterilization process micro-organisms have to be destroyed. The inactivation of microorganisms via electron treatment follows an exponential law. Thus, a finite probability of surviving microorganisms regardless of the magnitude of the absorbed sterilization dose can be calculated. The probability of surviving depends on number and types (i.e. species) of microorganisms present on the product (so called bioburden), the sterilization process lethality, and, in some cases, the environment (i.e. temperature, gas atmosphere) in which the organisms exist before, during and after treatment.

It follows that the sterility of individual items in a population of products sterilized cannot be ensured in the absolute sense. A sterility assurance level (SAL) is derived mathematically and it defines the probability of a viable microorganism on an individual product unit. Thus, more specifically, "sterile" means that a specific sterility assurance level (SAL) is achieved. For medical devices the sterility assurance level is defined in the DIN EN 556-1 and amounts to at least 10⁻⁶. That means if one million sterilized medical devices have been tested on sterility in average one non-sterile medical device has been detected. The same definition is applied in the present description with claims, *mutatis mutandis.*

Thus, it is an aim of the present invention to provide a bulk of a drug substance fulfilling the sterility assurance level defined in DIN EN 556-1.

### Step a)

In a first step of the method, a bulk of the substance, in particular a drug substance, is provided. Drug substances are typically produced via synthetic procedures, semi-synthetic procedures, microbiological fermentation procedures (e.g. by culturing of bacteria, yeast or other cells), or combination of the before-mentioned. Food products and food ingredients are typically obtained from agricultural processes, including farming. Chemicals are typically obtained from synthetic processes, including petrochemical processes.

In certain particularly interesting embodiments, the bulk of the substance, in particular a drug substance, is present in micronised form, i.e. in a particulate form where the mean particle size is in the range of 0.01-30 µm.

In many instances, the bulk of the substance has a weight of in the range of 1-40 kg, such as in the range of 5-25 kg.

### Step b)

In a second step of the method of the invention, the bulk is packed in a primary packing means and the primary packing means is subsequently sealed, whereby a package comprising said bulk of the substance is formed. It should be understood that the bulk which is actually packed may constitute a fraction of the bulk of the substance originally prepared.

The primary packing means is suitably in the form of a bag, a box, a container, flexible tube, barrel, tetra pack, etc. The material must - of course - be sufficiently thin in order to allow the electron beam effectively reach the bulk. In preferred embodiments, the primary packing means is in the form of a bag.

The primary packing means, in particular a bag, is typically made of a polymeric material such as polyethylene, polypropylene, polyvinylpropylene, or polyvinylchloride, in particular a polyethylene or polypropylene material with a gas barrier, such as a polyethylene material with a gas barrier.

Sealing of the primary package means is conducted so as to reduce the risk of oxidation of the substance while the package is exposed to the electron treatment as well as subsequently upon storage.

The void space in the package essentially consists of an unreactive gas, e.g. carbon dioxide, nitrogen, helium, argon, krypton, other inert gasses, etc. or mixtures thereof, in particular carbon dioxide. Alternatively, or more preferably combined with the presence of an unreactive gas, the package holds a partial vacuum. By the void space of the package is meant the free space around and between the substance particles, i.e. the space occupied by a gas which is in direct contact with the substance.

It should be understood that the bulk may be packed in a primary packing means (e.g. a bag) which is subsequently arranged in a secondary packing means (e.g. also a bag). It should also be understood that one or more primary packing means (e.g. 1-20) may be packed in a single secondary packing means, if desirable.

The secondary packing means may be of the same type as the primary packing means and is sealed subsequent to arrangement of the primary packing means therein.

In one preferred embodiment, (i) the bulk of the substance is packed in a primary packing means consisting of a bag of a polyethylene or polypropylene material, (ii) the primary packing means is sealed, (ii) the primary packing means (one or more primary packing means, in particular one) is arranged within a secondary packing means consisting of a bag of a polyethylene or polypropylene material, and (iv) the secondary packing means is sealed. The unreactive gas in the void space of the primary packing means is preferably carbon dioxide.

In order to increase safety and reduce the risk of subsequent rupture of the packing means and thereby damage of the substance, it is preferred that the primary packing means (and, if present, the secondary packing means) is at least partly enclosed within (preferably fully enclosed within), or wrapped in, a more rigid material, e.g. a paperboard, cardboard or plastic box or tray, upon handling and exposure to electron beams (see the Examples). Furthermore, such a box will force the bulk material in a well-defined shape. Particularly, the thickness is relevant for the result of the sterilisation process (see below).

The dimensions of the package play a certain role due to the fact that the electrons should be able to penetrate sufficiently deep into to bulk (see further below). The smallest dimension (typically corresponding to the thickness) is of course of particular relevance, and in preferred embodiments, the smallest dimension (the thickness) of the package is in the range of 0.3-20 cm, e.g. 0.5-10 cm, such as 1-7 cm.

The package most often has the form of a thin block where the ratio between the largest dimension (typically corresponding to the length) and the smallest dimension (typically the thickness) is at least 5:1, such as at least 7:1 or at least 10:1.

### Step c)

In the third step of the process, the package is exposed to electron treatment, thereby obtaining a package comprising a sterilized bulk of the substance, in particular a drug substance.

The package is typically exposed to electron treatment by placing the package in an electron beam field, or, preferably, to allow the package to pass through a field of electron beams. This energy source has one big advantage in that it can provide a high amount of energy in extremely short time. The typical time of exposure depends on the dose; however typically, the exposure time is about 1 second. In this way, micro-organisms can be inactivated with a smaller degradation of the carrier substance. The sterilization process via electrons is a physical one. The electron field is generated by an electron beam generator in combination with a so-called scanner. When properly applied, this process is a safe and reliable industrial one.

One particular advantage of the process is that the temperature increase of the bulk of the substance can be kept low. An absorbed dose of about 25 kGy is a typical one for the sterilization process of medical devices. Neglecting thermal conductivity and assuming a specific thermal capacity of 1 kJ x kg⁻¹ x K⁻¹ the electron treatment results in a temperature increase less than 25 Kelvin. Thus, the temperature during the sterilization process can be controlled in order to avoid temperature dependent degradation of the substance.

The absorbed dose of the electron beams is typically in the range of 10-40 kGy, e.g. in the range of 10-15 kGy (based on the bulk of the substance). As the dose depends on the type and the number of the micro-organisms and as the inactivation of the micro-organisms is a statistical process, the dose of the electron beams should be adjusted in accordance with the expected number of micro-organisms in the non-sterilized bulk of the substance.

Under conventional conditions (treatment in air at room temperature) for good results (concerning sterility), it is necessary to have an absorbed dose between 10 and 15 kGy. Such conditions will, however, lead to an undesirable degree of degradation of the substance. A lower dose exposure will not be sufficient to inactivate the micro-organisms.

Electrons generally have a low penetration depth controlling via their kinetic energy as well as the area density of the material to be sterilized. The maximum kinetic energy is limited to 10 MeV in order to avoid the generation of radioactive substances. The penetration depth of electron in a material with a density of 1 g cm⁻³ is showed in Figure 1.

For this reasons, the maximum thickness of the bulk in the package should be limited to 10 cm, such as 5 cm, (single side treatment) or 20 cm, such as 10 cm, (both side treatment). The heat conductivity of penicillines is very low, thus, if the bulk is too thick, the bulk will not be able to dissipate the heat. This leads to unwanted degradation of the drug substance.

In order to create a suitable balance between exposure time, absorbed dose, efficacy, and degradation, the package having an approximate form as a thin block and being filled with an unreactive gas is typically exposed to electron beams from both sides of the package material.

### A package comprising a sterilized drug

It is believed that at least some of the sterilized packages obtained by the method defined herein are hitherto unknown packages. Thus, the present invention also relates to a package comprising a sterilized drug substance in a sealed primary packing means, said drug substance being selected from penicillins, in particular from amoxicillin and ampicillin, the void space of primary packing means essentially consisting of an unreactive gas, in particular carbon dioxide.

The primary packing means preferably comprises a polyethylene or polypropylene bag, in particular a bag including a gas barrier.

In a particular embodiment, the primary packing means is a polyethylene or polypropylene bag which is packed in a secondary packing means, typically also a polyethylene or polyethylene bag. The package typically has a thickness of in the range of 0.3-20 cm, e.g. 0.5-10 cm, such as 1-7 cm.

The package is preferably prepared and sterilized as described above.

### EXAMPLES

### Example 1 - Electron treatment in carbon dioxide gas atmosphere

A bulk of amoxicillin was obtained from conventional production process. The bulk had a white colour. The number of viable micro-organisms in the bulk, i.e. bioburden, was determined to XXX per kg amoxicillin.

5-15 kg of the bulk of amoxicillin was loaded in a polyethylene bag with carbon dioxide gas, and the bag was evacuated and sealed. The bag was subsequently placed in second (similar) polyethylene bag, and the second bag was then sealed. The package consisting of the bags and the bulk of amoxicillin was arranged in a cardboard box so as to obtain a regular shape of the bulk, namely a thickness of about 7 cm, a width of about 60 cm and a length of about 80 cm.

The bag was exposed to electron beams (10 MeV) for at period of 1 second by simultaneous treatment on both sides, whereby the bulk of the amoxicillin absorbed 10 - 15 kGy.

The number of viable micro-organisms in the bulk of the sterilized amoxicillin was determined to less than XXX per kg amoxicillin. The bulk had maintained its white colour. Analytical results (XXX) showed a less than XXX% degradation of the amoxicillin.

### Example 2 - Electron treatment in air atmosphere

The procedure described in Example 1 was repeated with the exception that the carbon dioxide gas was replaced by atmospheric air.

The number of viable micro-organisms in the bulk of the sterilized amoxicillin was determined to less than XXX per kg amoxicillin. The bulk had a slightly brown colour. Analytical results (XXX) showed about XXX% degradation of the amoxicillin.

### Example 3 - Electron treatment in vacuum

The procedure described in Example 1 was repeated with the exception that the carbon dioxide gas was replaced by atmospheric air, but the polyethylene bag was evacuated before sealing.

The number of viable micro-organisms in the bulk of the sterilized amoxicillin was determined to less than XXX per kg amoxicillin. The bulk had an off-white colour. Analytical results (XXX) showed about XXX% degradation of the amoxicillin.

## Claims

1. A process for the preparation of a package comprising a sterilised bulk of a substance, said process comprising the following steps:
a) providing the bulk of the substance;
b) packing said bulk in a primary packing means and sealing said primary packing means, the void space in said primary packing means essentially consisting of an unreactive gas, thereby forming a package comprising said bulk of the substance; and
c) exposing said package to electron treatment, thereby obtaining a package comprising a sterilized bulk of the substance.

2. The process according to any one of the preceding claims, wherein the smallest dimension (the thickness) of the package is in the range of 0.3-20 cm, e.g. 0.5-10 cm, such as 1-7 cm.

3. The process according to any one of the preceding claims, wherein the absorbed dose of the electron treatment is in the range of 10-40 kGy, e.g. in the range of 10-15 kGy.

4. The process according to any one of the preceding claims, wherein the primary package means is a bag of a polymeric material, e.g. a polymeric material selected from polyethylene, polypropylene, polyvinyl propylene, or polyvinylchloride, in particular a polyethylene material with a gas barrier.

5. The process according to any one of the preceding claims, wherein the void space in the package essentially consists of carbon dioxide.

6. The process according to any one of the preceding claims, wherein the package holds a partial vacuum.

7. The process according to any one of the preceding claims, wherein the bulk of the drug substance is in a particulate form where the mean particle size is in the range of 0.01-30 µm.

8. The process according to any one of the preceding claims, wherein the substance is a drug substance, e.g. a drug substance selected from penicillins, in particular from amoxicillin and ampicillin.

9. A package comprising a sterilized drug substance in a sealed primary packing means, said drug substance being selected from penicillins, in particular from amoxicillin and ampicillin, the void space of primary packing means essentially consisting of an unreactive gas, in particular carbon dioxide.

10. The package according to claim 9, wherein the primary packing means comprising a polyethylene or polypropylene bag.
